# EUROPÄISCHE PATENTSCHRIFT

(11) **EP 2 780 309 B1**
(45) Veröffentlichungstag und Bekanntmachung des Hinweises auf die Patenterteilung: **07.10.2015**
(21) Anmeldenummer: 12783114.7
(22) Anmeldetag: 24.10.2012
(51) Int. Cl.: C07C 29/10, C07C 29/80, C07C 29/90, C07C 29/84, C07C 41/42, C07C 41/44, C07C 31/22, C07C 43/13, C07D 319/06

(54) **VERFAHREN ZUR GEWINNUNG VON DI-TRIMETHYLOLPROPAN UND MIT TRIMETHYLOLPROPAN ANGEREICHERTEN PRODUKTSTRÖMEN AUS DEN NEBENSTRÖMEN DER TRIMETHYLOLPROPANHERSTELLUNG**
METHOD FOR RECOVERING DI-TRIMETHYLOLPROPANE AND TRIMETHYLOLPROPANE-ENRICHED PRODUCT STREAMS FROM THE SIDE STREAMS OF TRIMETHYLOLPROPANE PRODUCTION
PROCÉDÉ D'OBTENTION DE DI-TRIMÉTHYLOLPROPANE ET DE FLUX DE PRODUITS ENRICHIS EN TRIMÉTHYLOLPROPANE À PARTIR DES FLUX DE SOUS-PRODUITS DE LA PRÉPARATION DE TRIMÉTHYLOLPROPANE

(30) Priorität: 19.11.2011 DE 102011118993
(43) Veröffentlichungstag der Anmeldung: 24.09.2014
(73) Patentinhaber: OXEA GmbH, 46147 Oberhausen (DE)
(72) Erfinder: FREY, Guido, D., 64560 Riedstadt (DE); SCHALAPSKI, Kurt, 46147 Oberhausen (DE); BERMANN, Dirk, 45475 Mülheim (DE); NOWOTNY, Norman, 45276 Essen (DE); STRUTZ, Heinz, 47445 Moers (DE); RAFF, Donald, K., Waynesville, NC 28786 (US)
(86) Internationale Anmeldenummer: PCT/EP2012/004440
(87) Internationale Veröffentlichungsnummer: WO 2013/072008

(56) Entgegenhaltungen:
- EP-A2- 1 178 030
- DE-A1- 19 840 276

## Beschreibung

Die vorliegende Erfindung betrifft ein Verfahren zur Gewinnung von Di-Trimethylolpropan und mit Trimethylolpropan angereicherten Produktströmen aus den Nebenströmen der Trimethylolpropanherstellung.

Trimethylolpropan ist ein dreiwertiger Alkohol, der für die Herstellung von Lacken, Polyurethanen und Polyestern, wie beispielsweise von Alkydharzen, von Bedeutung ist. Industriell wird Trimethylolpropan durch Kondensationsreaktion von n-Butyraldehyd mit Formaldehyd nach verschiedenen Varianten hergestellt.

Bei dem sogenannten Hydrierverfahren addiert man zumindest zwei Mole Formaldehyd mit einem Mol n-Butyraldehyd in Gegenwart einer katalytischen-Menge eines tertiären Amins über die Zwischenstufe Monomethylolbutyraldehyd zunächst zum Dimethylolbutyraldehyd, der anschließend in einem Hydrierschritt zum Trimethylolpropan umgesetzt wird. Nach dem in WO98/28253 A1 beschriebenen Verfahren wird Formaldehyd mit bis zu einem achtfachen molaren Überschuss eingesetzt. Das erhaltene Reaktionsgemisch aus dem Aldoladditionsschritt wird entweder destillativ oder durch Phasentrennung aufgearbeitet. Bei der destillativen Aufarbeitung werden nicht umgesetzte oder teilumgesetzte Ausgangsverbindungen als flüchtige Komponenten abgezogen und in die Reaktionsstufe zurückgeführt, während das Sumpfprodukt weiter umgesetzt wird. Wird anstelle der destillativen Aufarbeitung das Reaktionsgemisch in einem Phasentrenner in die wässrige und organische Phase geschieden, wird die organische Phase in die Aldoladdition zurückgefahren und die wässrige Phase weiter verarbeitet. Es folgt eine katalytische und/oder thermische Behandlung, um Monomethylolbutyraldehyd in Dimethylolbutyraldehyd zu überführen. Hierbei gebildete Nebenprodukte werden destillativ abgetrennt und das Sumpfprodukt dieser Destillation wird anschließend katalytisch hydriert, um Trimethylolpropan zu gewinnen. Das erhaltene rohe Trimethylolpropan wird anschließend einer Reindestillation unterzogen. Nach Leicht- und Mittelsiederabtrennung erhält man gereinigtes Trimethylolpropan als Zwischenfraktion, während höhersiedende Kondensationsprodukte, in denen Trimethylolpropanäquivalente gebunden sind, als Nachlauf- oder Sumpffraktion anfallen.

Neben dem Hydrierverfahren wird Trimethylolpropan technisch auch über die sogenannte Cannizzaro-Reaktion hergestellt. In einer ersten Reaktionsstufe lässt man dabei n-Butyraldehyd und Formaldehyd unter Zugabe von stöchiometrischen Mengen einer Base zum Dimethylolbutyraldehyd reagieren, das anschließend mit überschüssigem Formaldehyd zum Trimethylolpropan reduziert wird, während gleichzeitig ein Äquivalent Formiat gebildet wird. Üblicherweise wird als Base eine wässrige Lösung einer Alkalimetall- oder Erdalkalimetallverbindung verwendet, beispielsweise Natrium-, Kalium- oder Calciumhydroxid. Da ein Äquivalent Alkalimetall- oder Erdalkalimetallformiat bei dem Cannizzaro-Verfahren als Koppelprodukt anfällt, hängt die Wirtschaftlichkeit dieser Verfahrensvariante auch von den Vermarktungschancen dieses Koppelprodukts ab. Die Aufarbeitung der erhaltenen, wässrigen Reaktionslösung, die Trimethylolpropan, Alkalimetall- oder Erdalkalimetallformiat und überschüssige Base enthält, erfolgt im Allgemeinen durch Extraktion. Nach Neutralisation der überschüssigen Base wird die wässrige Lösung mit einem organischen Lösungsmittel, beispielsweise mit Ethylacetat extrahiert. Die organische Phase wird von der wässrigen Phase, die die Alkalimetall- oder Erdalkalimetallformiate gelöst enthält, getrennt und nach Entfernung des Extraktionsmittels wird Trimethylolpropan durch Destillation gewonnen. Das erhaltene Trimethylolpropan kann weiteren Reinigungsverfahren unterzogen werden. Nach US 5,603,835 wird zunächst aus erhaltenem Trimethylolpropan eine wässrige Lösung hergestellt, die nochmals mit einem organischen Lösungsmittel, beispielsweise mit Methyl-tertiär-butylether, extrahiert wird. Aus der erhaltenen wässrigen Lösung wird Trimethylolpropan mit einer verbesserten Farbzahl von weniger als 100 APHA Einheiten gewonnen.

Gemäß dem aus US 5,948,943 bekannten Verfahren wird die nach der Cannizzaro-Reaktion erhaltene wässrige, rohe Reaktionslösung bei einer solchen Temperatur mit einem geeigneten organischen Lösungsmittel behandelt, dass nur eine flüssige Phase das Extraktionsgefäß verlässt. Bei der anschließenden Abkühlung außerhalb des Extraktionsgefäßes trennt sich die wässrige von der organischen Phase und aus der wässrigen Phase kann Trimethylolpropan mit einer Farbzahl von weniger als 100 APHA isoliert werden.

Es ist ebenfalls bekannt, die Cannizzaro-Reaktion mit einer organischen Base, beispielsweise mit einem tertiären Amin, durchzuführen. Nach der aus WO97/17313 A1 bekannten Arbeitsweise wird Formaldehyd mit n-Butyraldehyd in Gegenwart von stöchiometrischen Mengen eines tertiären Amins hergestellt, wobei ein Äquivalent des Ammoniumformiats entsteht. Anschließend wird aus dem Rohgemisch Wasser, überschüssiges tertiäres Amin und überschüssiger Formaldehyd abgetrennt und das verbleibende Gemisch erhitzt. Dabei wird das Ammoniumformiat in das tertiäre Amin und Ameisensäure gespalten, wobei das tertiäre Amin und weitere flüchtige Bestandteile abgetrennt werden und es zur Bildung von Trimethylolpropanformiat kommt. Das abgetrennte tertiäre Amin wird entweder in die Cannizzaro-Stufe zurückgeführt oder als Katalysator für die Umesterung des Trimethylolpropanformiats in einer nachgeschalteten Reaktion mit einem zugesetzten niedrigen aliphatischen Alkohol verwendet. Das dabei freigesetzte Trimethylolpropan wird anschließend aus dem Rohprodukt isoliert.

Unabhängig davon, ob die Herstellung von Trimethylolpropan nach dem Hydrierverfahren unter Verwendung katalytischer Mengen eines tertiären Amins, nach dem Cannizzaro-Verfahren mit molaren Mengen eines tertiären Amins und nachfolgender Umesterung des gebildeten Trimethylolpropanformiats oder nach dem Cannizzaro-Verfahren mit molaren Mengen von Alkalimetall- oder Erdalkalimetallhydroxiden und ihrer extraktiven Abtrennung erfolgt, wird das erhaltene rohe Trimethylolpropan einer ein- oder mehrfachen destillativen Reinigung unterzogen, die aufgrund des hohen Siedepunkts im Unterdruck erfolgt. Nach DE 100 58 303 A1 wird die destillative Aufarbeitung des Trimethylolpropans mit einer Ionenaustauscherbehandlung kombiniert, wobei entweder der Aldolisierungsaustrag oder der Hydrieraustrag vor der destillativen Aufarbeitung mit einem stark basischen Ionenaustauscher in Kontakt gebracht wird.

Aus DE 1 768 348 B ist bekannt, zwei verschiedene Aldehyde, beispielsweise Acetaldehyd und Butyraldehyd mit Formaldehyd in einem wässrigen, alkalischen Medium umzusetzen. Das erhaltene Reaktionsgemisch wird zunächst durch Säurezugabe neutralisiert, von suspendierten Feststoffen befreit und anschießend mit sauren und basischen Ionenaustauschern behandelt.

Bei der destillativen Reinigung fallen schwersiedende Fraktionen mit einem im Vergleich zum Trimethylolpropan höheren Siedepunkt oder Rückstände an, in denen Derivate des Trimethylolpropans vorliegen und aus diesem durch Reaktion mit beispielsweise Methanol, Formaldehyd oder auch mit einem weiteren Molekül Trimethylolpropan in den vorgeschalteten Reaktionen entstanden sind. Unter diesen Derivaten sind besonders formaldehydhaltige Acetale vertreten, die durch das Strukturelement -O-CH₂-O- charakterisiert werden und auch als Formale aufgefasst werden können. Unter den Formalen können folgende lineare und zyklische Formale des Trimethylolpropans strukturell beschrieben werden:
Monozyklisches Formal des Trimethylolpropans: 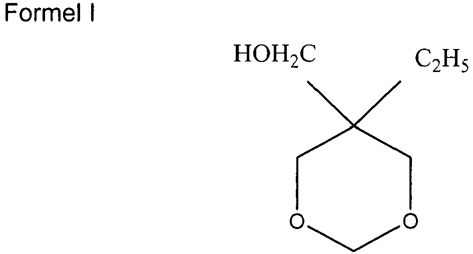
Lineares bis-Trimethylolpropan-Formal:

   Formel II [C₂H₅C(CH₂OH)₂CH₂O]₂CH₂
Methyl-(monolineares) Formal des Trimethylolpropans:

   Formel III C₂H₅C(CH₂OH)₂CH₂OCH₂OCH₃
Methyl-(bis-lineares) Formal des Trimethylolpropans:

   Formel IV C₂H₅C(CH₂OH)₂CH₂OCH₂OCH₂OCH₃

Hierbei siedet das monozyklische Formal des Trimethylolpropans (I) bei einer niedrigeren Temperatur als Trimethylolpropan selbst. Die vom Methanol abgeleiteten Formale (III) und (IV) haben einen mit Trimethylolpropan vergleichbaren Siedepunkt während das lineare bis-Trimethylolpropan-Formal (Formel II) als schwersiedende Komponente vorliegt. Darüber hinaus sind noch weitere lineare und zyklische sauerstoffhaltige Verbindungen, wie das zyklische Formal des Di-Trimethylolpropans 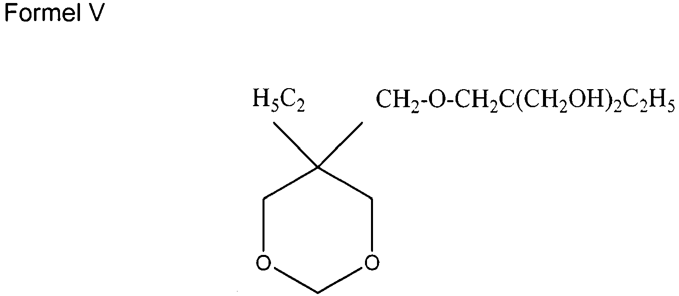 in den Destillationsrückständen zugegen.

Ebenfalls sind in den schwersiedenden Fraktionen und Rückständen der destillativen Aufarbeitung des rohen Trimethylolpropans noch substantielle Mengen an Di-Trimethylolpropan [C₂H₅C(CH₂OH)₂-CH₂-]₂-O und Trimethylolpropan selbst enthalten. Darüber hinaus sind leichtsiedende Komponenten, wie Methanol oder 2-Ethyl-2-Methyl-1,3-Propandiol in geringen Mengen anwesend.

Da in den schwersiedenden Fraktionen und Rückständen der destillativen Aufarbeitung des Trimethylolpropans erhebliche Mengen an Derivaten vorliegen, in denen Äquivalente des Trimethylolpropans chemisch gebunden sind, werden eine Reihe von Verfahren vorgeschlagen, um insbesondere formaldehydhaltige Acetale zu spalten und Trimethylolpropan freizusetzen, um auf diese Weise die Ausbeute des gesamten Trimethylolpropanherstellprozesses zu verbessern. Nach WO 2004/013074 A1 werden die bei der Trimethylolpropanherstellung erhaltenen schwersiedenden Fraktionen sowie Destillationsrückstände mit Säure behandelt, wobei der Wassergehalt im Reaktionsansatz 20-90 Gew.-% betragen soll. Aus dem säurebehandelten Produkt kann entweder Trimethylolpropan destillativ gewonnen werden oder das behandelte Produkt kann in die Hydrierstufe des Dimethylolbutyraldehyds zum Trimethylolpropan zurückgeführt werden. Die hydrierende Spaltung von linearen oder zyklischen Acetalen in wässrigen Lösungen in Gegenwart eines heterogenen Hydrierkatalysators zu dem gewünschten mehrwertigen Alkohol ist aus DE 198 40 276 A1 bekannt. Das Verfahren erfordert Hydriertemperaturen oberhalb von 160°C, um den schädlichen Einfluss von Formiaten, die besonders bei dem Arbeiten nach dem Cannizzaro-Verfahren noch zugegen sein können, auf die Hydrierleistung des Katalysators zurückzudrängen. Die hydrierende, katalytische Spaltung kann ebenfalls in Gegenwart einer Säure, beispielsweise in Gegenwart einer niederen Carbonsäure oder sauer reagierender Feststoffe durchgeführt werden.

Die schwersiedenden Fraktionen und die Rückstände der destillativen Aufarbeitung der Trimethylolpropanherstellung enthalten neben den zuvor erwähnten formaldehydhaltigen Acetalen auch bedeutende Mengen an Di-Trimethylolpropan, das ebenfalls als wertvolles Ausgangsmaterial zur Herstellung von Alkydharzen, Weichmachern und Schmiermitteln technische Bedeutung besitzt. Aus dem Stand der Technik sind Verfahren bekannt, Di-Tri-methylolpropan aus diesen Rückständen zu gewinnen und so erhaltenes Produkt bei Bedarf weiter zu reinigen.

Nach DE 2058518 A1 wird der Di-Trimethylolpropan haltige Destillationsrückstand einer Wasserdampfdestillation mit überhitztem Wasserdampf unter reduziertem Druck unterzogen. Aus dem erhaltenen wässrigen Destillat wird nach Wasserabtrennung Di-Trimethylolpropan erhalten, das im Bedarfsfalle aus einem organischen Lösungsmittel, beispielsweise Aceton, umkristallisiert werden kann.

EP 1 178 030 A2 behandelt ein Verfahren zur Gewinnung von Di-Trimethylolpropan aus den Destillationsrückständen der Trimethylolpropanherstellung. Die Destillationsrückstände wurden mit einer Säure und gegebenenfalls mit einem Hydroxylaminsalz behandelt und anschließend destillativ aufgearbeitet. Di-Trimethylolpropan wird an einem Fallfilm-Verdampfer als Destillat abgezogen.

Da die destillative Reinigung von Di-Trimethylolpropan infolge des hohen Siedepunktes nur sehr schwer möglich ist und aufgrund der hohen anzuwendenden Temperaturen auch die Zersetzung des Di-Trimethylolpropans zu befürchten ist, wird auch die direkte Aufarbeitung des Destillationsrückstands durch Umkristallisation zur Gewinnung von Di-Trimethylolpropan beschrieben. DE 2358297 A1 behandelt die einfache Kristallisation einer wässrigen Lösung des Destillationsrückstands, wobei in der wässrigen Lösung die Salzkonzentration auf ein besonderes Verhältnis eingestellt wird, um das Ausfällen von Di-Trimethylolpropan in ausreichender Reinheit zu ermöglichen. Wenn Trimethylolpropan nach dem Cannizzaro-Verfahren hergestellt wird, kann der Salzgehalt, beispielsweise der Alkalimetallformiatgehalt in dem Destillationsrückstand bereits genügend hoch sein, um das Ausfallen von Di-Trimethylolpropankristallen in befriedigenderweise nach Auflösung in Wasser sicherzustellen. Gegebenenfalls ist der wässrigen Lösung ein weiteres Salz zuzusetzen, beispielsweise ein Alkalimetallsalz.

US 2004/0254405 A1 offenbart ein Verfahren zur Umkristallisation des Destillationsrückstands unter Verwendung organischer Lösungsmittel, beispielsweise Aceton oder Methylethylketon, wobei die Kristallisationstemperatur, die Lösungsmittelmenge und der Gehalt an Di-Trimethylolpropan im Destillationsrückstand in besonderer Weise einzuhalten sind. Die Verwendung eines Gemisches aus einem geeigneten Lösungsmittel und Wasser für die Isolierung von Di-Trimethylolpropan aus den Destillationsrückständen der Trimethylolpropanherstellung wird in DE 10 2008 038 021 A1 beschrieben. Man erhält zunächst eine organische Lösungsmittelphase und einen viskosen Rückstand, trennt die Phasen und extrahiert die organische Lösungsmittelphase mit Wasser. Die Wasserphase wird abgetrennt und enthaltene Lösungsmittelreste werden entfernt. Aus der verbleibenden Wasserphase wird Di-Trimethylolpropan kristallisiert.
In der DE 10 2010 033 844 A1 wird ebenfalls ein Verfahren zur Gewinnung von Di-Trimethylolpropan aus den Nebenströmen der Trimethylolpropanherstellung behandelt. Dabei werden die anfallenden schwersiedenden Fraktionen und Rückstände in Wasser aufgelöst und die wässrige Lösung wird in Gegenwart einer aciden Verbindung unter Spaltung von formaldehydhaltigen Acetalen katalytisch hydriert. Das wässrige Hydriergut wird nach Feststoffabtrennung anschließend sowohl mit basischen als auch mit sauren Ionenaustauschern in Kontakt gebracht. Aus dem wässrigen Eluat wird ein mit Trimethylolpropan angereicherter Produktstrom abdestilliert und Di-Trimethylolpropan verbleibt als Destillationsrückstand. Damit Di-Trimethylolpropan in ausreichender Qualität im Destillationsrückstand anfällt, ist nach dem Prozess gemäß der DE 10 2010 033 844 A1 die Behandlung des wässrigen Hydrierguts sowohl mit basischen als auch mit sauren Ionenaustauschern zwingend notwendig.

Die bekannten Verfahren zur Gewinnung von Di-Trimethylolpropan aus schwersiedenden Fraktionen und Rückständen, die einen höheren Siedepunkt als Trimethylolpropan aufweisen und die bei der destillativen Aufarbeitung im Rahmen der Trimethylolpropanherstellung anfallen, erfordern entweder aufwendige Umkristallisationsschritte oder eine aufwendige Wasserdampfdestillation mit der nachfolgenden Wasserentfernung aus dem Wasserdampfdestillat.

Auch wird bei Verfahren, bei denen Di-Trimethylolpropan als Destillationsrückstand anfällt, Di-Trimethylolpropan nicht immer in ausreichender Qualität erhalten, um es in möglichst vielen technischen Anwendungen einzusetzen. Zudem ist vor der Destillationsstufe eine Reinigung mit Ionenaustauschern notwendig, um den Gehalt an Verunreinigungen im Destillationsrückstand möglichst gering zu halten.

Es besteht daher ein Bedarf, Di-Trimethylolpropan aus solchen schwersiedenden Fraktionen und Rückständen möglichst einfach mit einer solchen Reinheit zu gewinnen, die für die vorgesehen technischen Anwendungen gefordert wird. Dabei sollen ebenfalls in diesen Fraktionen und Rückständen noch physikalisch eingemischtes Trimethylolpropan sowie darin enthaltene Derivate mit chemisch gebundenen Trimethylolpropaneinheiten als trimethyl-olpropanreiche Fraktion isoliert werden, die wieder in den Trimethylolpropanreinigungsprozess zurückgeführt werden kann, so dass neben der Gewinnung von reinem Di-Trimethylolpropan auch die Ausbeute an Trimethylolpropan über den gesamten Herstellprozess verbessert werden kann. Auf diese Weise sollen die schwersiedenden Fraktionen und Rückstände, die bei der destillativen Aufarbeitung im Rahmen der Trimethylolpropanherstellung anfallen, möglichst wirtschaftlich genutzt werden.

Die vorliegende Erfindung betrifft daher ein Verfahren zur Gewinnung von Di-Trimethylolpropan und mit Trimethylolpropan angereicherten Produktströmen aus den schwersiedenden Fraktionen und Rückständen, die bei der destillativen Reinigung von Trimethylolpropan anfallen. Es ist dadurch gekennzeichnet, dass man:
(a) diese schwersiedenden Fraktionen und Rückstände vereinigt und unter Zusatz eines polaren Lösungsmittels eine Lösung herstellt;
(b) die nach Schritt a) hergestellte Lösung bei einer Temperatur von 160 bis 280°C und bei einem Druck von 1 bis 30 MPa mit Wasserstoff in Gegenwart eines Katalysators und einer aciden Verbindung behandelt;
(c) die nach Schritt b) erhaltene Lösung vom Katalysator und weiteren Feststoffen, falls vorhanden, abtrennt;
(d) die nach Schritt c) erhaltene Lösung in einer ersten Destillationseinrichtung in eine Kopffraktion enthaltend das polare Lösungsmittel und Leichtsieder und in eine Sumpffraktion mit einem Gehalt an dem polaren Lösungsmittel bis 5000 Gew.-ppm, bezogen auf die Sumpffraktion, auftrennt;
(e) die nach Schritt d) erhaltene Sumpffraktion auf eine zweite Destillationseinrichtung mit mindestens 5 theoretischen Böden gibt, die als Dünnschichtverdampfer mit Kolonnenaufsatz ausgestaltet ist, und eine mit Trimethylolpropan angereicherte Kopffraktion abzieht und eine Sumpffraktion entnimmt; und
(f) die nach Schritt e) erhaltene Sumpffraktion auf eine dritte Destillationseinrichtung mit mindestens 4 theoretischen Böden, die als Dünnschichtverdampfer mit Kolonnenaufsatz ausgestaltet ist, gibt, in der Di-Trimethylolpropan als Kopffraktion gewonnen wird und Hochsieder als Sumpffraktion entfernt werden.

Ausgangsprodukte für das erfindungsgemäße Verfahren sind Produktströme, die bei der destillativen Reinigung von Trimethylolpropan anfallen und einen höheren Siedepunkt als Trimethylolpropan aufweisen und als schwersiedende Fraktionen bezeichnet werden können.

Neben diesen schwersiedenden aber bei der Destillation noch flüchtigen Komponenten kommt auch der verbleibende Destillationsrückstand in dem erfindungsgemäßen Verfahren zum Einsatz. Diese vereinigten Produktströme enthalten als Hauptkomponenten noch physikalisch eingemischtes Trimethylolpropan, im Allgemeinen in einem Bereich von 5 bis 30 Gew.-%, Di-Trimethylolpropan, im Allgemeinen in einem Bereich von 10 bis 35 Gew.-% sowie das lineare bis-Trimethylolpropan-Formal in einem Bereich von 25 bis 70 Gew.-%, bezogen auf die gesamte Einsatzmasse. Weitere identifizierte Produkte sind 2-Ethyl-2-Methyl-1,3-Propandiol und das monozyklische Formal des Trimethylolpropans, die aufgrund ihres vergleichsweise geringen Siedepunktes nur noch in geringen Mengen bis üblicherweise bis zu 3 Gew.-% anwesend sind. Zyklische und lineare Formale darunter das Methyl-(monolineare) Formal des Trimethylolpropans (III), das Methyl-(bis-lineare) Formal des Trimethylolpropans (IV) sowie das zyklische Formal des Di-Trimethylolpropans (V) bilden den Rest der organischen Komponenten in dem Gemisch.

Unabhängig davon, ob Trimethylolpropan nach dem Cannizzaro-Verfahren unter Verwendung von Alkalimetall- oder Erdalkalimetallverbindungen hergestellt wird oder nach dem Hydrierverfahren in Gegenwart katalytischer Mengen von Trialkylaminen oder nach dem Cannizzaro-Verfahren unter Verwendung stöchiometrischer Mengen an Trialkylaminen produziert wird, können die schwersiedenden Fraktionen und die Rückstände, die bei der destillativen Reinigung von Trimethylolpropan nach dem jeweiligen Herstellverfahren anfallen, gemäß der erfindungsgemäßen Arbeitsweise aufgearbeitet werden. Darüber hinaus kann das Einsatzgemisch noch Alkalimetall- oder Erdalkalimetallformiate enthalten, deren Gehalte in Abhängigkeit von der Art des für die Herstellung von Trimethylolpropan angewandten Verfahrens schwanken.

Die schwersiedenden Fraktionen, die einen höheren Siedepunkt als Trimethylolpropan aufweisen, und die Rückstände aus der Destillation von Trimethylolpropan werden vereinigt und mit einem polaren Lösungsmittel unter Bildung einer Lösung versetzt. Als polares Lösungsmittel eignet sich ein niederer C₁-C₅ aliphatischer Alkohol oder C₂-C₁₀-Dialkylether, wie Methanol, Ethanol, Propanol oder Diethylether, oder insbesondere Wasser. Im Allgemeinen stellt man eine Lösung mit einem Gehalt an organischen Komponenten ohne Berücksichtigung des polaren Lösungsmittels von 30 bis 90 Gew.-%, vorzugsweise von 50 bis 80 Gew.-% her, bezogen auf die Gesamtmasse. Geringere Gehalte an organischen Komponenten sind aufgrund des hohen Lösungsmittelanteils nicht zweckmäßig, bei zu hohen Gehalten ist besonders bei Raumtemperatur mit Inhomogenitäten in der Lösung oder mit dem Ausfallen von Feststoffen zu rechnen. Zweckmäßigerweise wird die Lösung bei einer Temperatur von über 50°C hergestellt. Ein Temperaturbereich für die Lösung, insbesondere für die wässrige Lösung, von 60°C bis 80°C ist vorzugsweise einzustellen.

Die erhaltene Lösung wird anschließend bei erhöhter Temperatur und erhöhtem Druck mit Wasserstoff in Gegenwart eines Katalysators und einer aciden Verbindung behandelt. Man arbeitet bei Temperaturen im Bereich von 160 bis 280°C, vorzugsweise 180 bis 230°C und bei Drücken im Bereich von 1 bis 30 MPa, vorzugsweise 5 bis 20 MPa. Bei den anwesenden aciden Verbindungen kann es sich um protische anorganische Säuren, organische Säuren oder um saure Feststoffe handeln. Als protische anorganische Säuren kommen Phosphorsäure oder Schwefelsäure in Betracht als organische Säuren niedere Carbonsäuren wie Ameisensäure, Essigsäure, Propionsäure oder die isomeren Buttersäuren.

Ihre Menge wird so bemessen, dass die der Hydrierung zu unterziehende Lösung einen pH-Wert im Bereich von 1 bis 5, vorzugsweise von 2 bis 4 aufweist.

Aufgrund der leichten Abtrennbarkeit ist jedoch das Arbeiten mit sauer reagierenden Feststoffen als acide Verbindung bevorzugt. Als solche Feststoffe eignen sich beispielsweise oxidische Verbindungen wie saures Aluminiumoxid, natürliche oder silikatische Stoffe, wie Mordenit, Montmorillonit oder saure Zeolithe, beispielsweise solche vom Y-Typ, die in technischen Mengen zur Verfügung stehen und beispielsweise bei der katalytischen Spaltung von Rohölen industriell eingesetzt werden. Ihr Zusatz richtet sich nach ihrer Acidität und je 100 Gew.-Teile Lösung werden sie im Allgemeinen in einer Menge von 0,5 bis 2, vorzugsweise von 0,5 bis 1,0 Gew.-Teilen verwendet, wobei die eingesetzten Mengen umso geringer sind je acider der Feststoff ist. Dabei stellt sich in der Lösung im Allgemeinen ein pH-Wert von 1 bis 6, vorzugsweise von 2 bis 4 ein.

Auch kommerziell verfügbare saure Ionenaustauscher, beispielsweise stark saure Ionenaustauscher wie Amberlyst 15, Amberlite IR 120, Amberlyst DPT-1, Dowex Marathon-C, Dowex HCR, Lewatit S 100 oder Naphion oder schwach saure Ionenaustauscher wie Amberlite ICR 86 oder Lewatit CNP können eingesetzt werden. Ihr Zusatz richtet sich nach ihrer Acidität und sie werden im Allgemeinen in einer Menge von 1 bis 20, vorzugsweise von 5 bis 10 Gew.-Teilen, bezogen auf 100 Gew.-Teile Lösung, verwendet, wobei die verwendeten Mengen umso geringer sind je acider der Feststoff ist.

Als Katalysatoren für den Hydrierschritt verwendet man übliche Hydrierkatalysatoren, wobei heterogenen Hydrierkatalysatoren der Vorzug gegeben wird, da sie auf einfache Weise vom Reaktionsgemisch abgetrennt werden können, beispielsweise bei der Suspensionshydrierung durch einfache Filtration. Auch bei fest angeordneten Katalysatoren, beispielsweise bei der Riesel- oder Sumpffahrweise, kann das Reaktionsgemisch vom Hydrierkatalysator leicht separiert werden.

Übliche Hydrierkatalysatoren enthalten als wirksame Komponenten ein Edelmetall aus der Reihe Ru, Rh, Pd oder Pt oder ein Übergangsmetall aus der Reihe Cu, Cr, Co, Ni, Fe und darunter insbesondere Raney-Katalysatoren und Chromit-Katalysatoren. Neben ungeträgerten Katalysatoren kommen auch Trägerkatalysatoren zum Einsatz, insbesondere sind für Ru, Rh, Pd oder Pt geeignete Trägermaterialien Aktivkohle, Aluminiumoxid, SiO₂, TiO₂, ZrO₂ sowie Silikate. Die Metallbeladung bei geträgerten Katalysatoren liegt üblicherweise im Bereich von 0,1 bis 15, vorzugsweise von 0,5 bis 10 und insbesondere von 1 bis 5 Gew.-%. Als besonders geeignet haben sich Ru, Pd und Pt auf Aktivkohle erwiesen.

Die Hydrierstufe wird in Gegenwart der aciden Verbindung, die entweder gelöst vorliegt, beispielsweise im Falle von zugesetzten anorganischen Säuren oder niederen organischen Carbonsäuren, oder die als in der Lösung suspendierter Feststoff zugegen ist, kontinuierlich oder diskontinuierlich durchgeführt, beispielsweise an fest angeordneten Katalysatoren nach der Rieselfahrweise oder Sumpffahrweise sowie unter Rühren nach der Suspensionshydrierung.

Bei der kontinuierlichen Fahrweise hat sich eine Katalysatorbelastung V/Vh, ausgedrückt in Durchsatzvolumen pro Katalysatorvolumen und Zeit von 0,1 bis 1 h⁻¹, vorzugsweise von 0,2 bis 0,5 h⁻¹, als zweckmäßig erwiesen. Bei der diskontinuierlichen Verfahrensführung verwendet man, bezogen auf 100 Gew.-Teile Einsatzlösung ohne Berücksichtigung der aciden Verbindung, von 0,1 bis 10, vorzugsweise von 0,5 bis 5 Gew.-Teile an Katalysator.

Nach beendeter Hydrierung wird das flüssige Reaktionsgemisch von Feststoffen befreit, beispielsweise durch Filtration. Dadurch wird vom Hydrierkatalysator und gegebenenfalls von weiteren Feststoffen abgetrennt, wenn in Gegenwart fester acider Verbindungen hydriert wurde. Wird die Hydrierung in Gegenwart gelöster acider Verbindungen durchgeführt, empfiehlt sich eine Neutralisation mit einer Base, bevor das Hydriergut weiter aufgearbeitet wird.

Das von Feststoffen befreite flüssige Hydriergut wird anschließend destillativ aufgearbeitet. Zunächst werden in einer ersten Destillationseinrichtung das polare Lösungsmittel und Leichtsieder, insbesondere Wasser und Methanol, das durch Hydrierung des bei der Acetalspaltung freigesetzten Formaldehyds entstanden ist, als Kopffraktion abgetrennt. Für die Abtrennung des polaren Lösungsmittels und der Leichtsieder eignen sich übliche Destillationseinrichtungen, wie eine Destillationskolonne mit Sumpfblase, die beispielsweise 2 bis 40 theoretische Böden aufweist, ein Dünnschichtverdampfer, ein Kurzwegverdampfer oder ein Abdampfbehälter, die üblicherweise bei Sumpftemperaturen von 100 bis 180°C und bei Normaldruck oder zweckmäßigerweise im Unterdruck bis zu 70 hPa betrieben werden. Der Zulauf zur ersten Destillationseinrichtung kann bei Raumtemperatur aufgegeben werden, vorteilhafterweise besitzt der Zulauf jedoch eine Temperatur von 50 bis 130°C, insbesondere 80 bis 120°C. Durch die Aufgabe des Zulaufs, der bereits eine erhöhte Temperatur aufweist, kann das abzutrennende polare Lösungsmittel schlagartig verdampfen und über die Kopffraktion abgeführt werden. Die erste Destillationseinrichtung wird dabei so betrieben, dass der Gehalt an dem polaren Lösungsmittel in der Trimethylolpropan und Di-Trimethylolpropan enthaltenden Sumpffraktion bis maximal 5000 Gew.-ppm, vorzugsweise bis 1000 Gew.-ppm und insbesondere bis 500 Gew.-ppm, bezogen auf die Masse der Sumpffraktion, liegt. Das Einhalten einer maximalen Grenze für den Lösungsmittelgehalt in der Sumpffraktion wirkt sich vorteilhaft auf die nachfolgende destillative Reinigung aus. Diese Sumpffraktion wird aus der ersten Destillationseinrichtung abgeführt und auf eine zweite Destillationseinrichtung gegeben. Insbesondere lässt sich Wasser als polares Lösungsmittel in der ersten Destillationseinrichtung abtrennen.

In der zweiten Destillationseinrichtung gewinnt man als Kopffraktion einen mit Trimethylolpropan angereicherten Produktstrom mit einem Gehalt in der Größenordnung im Bereich von 90 bis 98 Gew.-% Trimethylolpropan, der zudem noch Zwischenläufe und Reste des polaren Lösungsmittels, wie Wasser und Leichtsieder enthält. Dieser Produktstrom kann in die Reinigungsstufe des Gesamtprozesses für die Herstellung von Trimethylolpropan zurückgeführt werden, zweckmäßigerweise in die Reindestillationsstufe zur Gewinnung von Trimethylolpropan. Die Abtrennung der Kopffraktion erfolgt an einer Destillationseinrichtung, die mindestens 5 theoretische Böden, vorzugsweise mindestens 8 theoretische Böden und insbesondere 8 bis 20 theoretische Böden aufweist und als Dünnschichtverdampfer mit Kolonnenaufsatz ausgestaltet ist. Ebenfalls ist in der zweiten Destillationseinrichtung die thermische Belastung möglichst gering zu halten und es ist bei möglichst geringen Verweilzeiten zu arbeiten. Dabei beträgt die Verweilzeit in der zweiten Destillationseinrichtung, also in der gesamten Destillationsapparatur im Allgemeinen von 2 bis 60, vorzugsweise von 10 bis 30 Sekunden. Als Kolonnenaufsatz eignen sich konventionelle Füllkörper- oder Packungskolonnen, vorzugsweise Strukturpackungskolonnen. Solche Packungen sind kommerziell erhältlich, beispielsweise als Montz- oder Sulzerpackungen. Auch die in dem erfindungsgemäßen Verfahren einzusetzenden Dünnschichtverdampfer sind fachübliche Aggregate, die kommerziell zur Verfügung stehen. Nicht geeignet sind eine Sumpfblase mit Kolonnenaufsatz oder ein Kurzwegverdampfer, da bei dieser Anordnung die Verweilzeit in der Destillationseinrichtung entweder zu hoch ist oder die Trennleistung nicht ausreicht. Die zweite Destillationseinrichtung wird im Allgemeinen bei Sumpftemperaturen von 210 bis 280°C und bei einem Druck von 2 bis 10 hPa betrieben. Die Sumpffraktion aus der zweiten Destillationseinrichtung wird anschließend auf eine dritte Destillationseinrichtung gegeben. Die dritte Destillationseinrichtung kann auch als Nachlaufabtrenneinrichtung angesehen werden und dient zur Gewinnung von Di-Trimethylolpropan in ausreichender Qualität. Di-Trimethylolpropan wird als Kopffraktion abgetrennt und Hochsieder werden als Sumpffraktion aus der dritten Destillationseinrichtung entfernt. Um Di-Trimethylolpropan in ausreichender Qualität zu erhalten, hat die dritte Destillationseinrichtung mindestens 4 theoretische Böden und insbesondere 4 bis 20 theoretische Böden aufzuweisen. Ebenfalls ist auch in der dritten Destillationskolonne die thermische Belastung möglichst gering zu halten und es ist bei möglichst geringen Verweilzeiten zu arbeiten. Dabei beträgt die Verweilzeit der Kopffraktion in der dritten Destillationseinrichtung im Allgemeinen von 1 bis 30, vorzugsweise von 5 bis 20 Sekunden. Als Anlagenschaltung verwendet man ebenfalls einen Dünnschichtverdampfer mit Kolonnenaufsatz, der die erforderliche Mindestanzahl an theoretischen Böden besitzt. Als Kolonnenaufsatz eignen sich konventionelle Füllkörper- oder Packungskolonnen, vorzugsweise Strukturpackungskolonnen. Solche Packungen sind kommerziell erhältlich, beispielsweise als Montz- oder Sulzerpackungen. Auch die in dem erfindungsgemäßen Verfahren einzusetzenden Dünnschichtverdampfer sind fachübliche Aggregate, die kommerziell zur Verfügung stehen. Nicht geeignet sind eine Sumpfblase mit Kolonnenaufsatz oder ein Kurzwegverdampfer, da bei dieser Anordnung die Verweilzeit des Kopfproduktes in der Destillationseinrichtung entweder zu hoch ist oder die Trennleistung nicht ausreicht. Die dritte Destillationseinrichtung wird im Allgemeinen bei Sumpftemperaturen von 240 bis 280 und bei einem Druck von 0,2 bis 5 hPa betrieben. Eine Sumpftemperatur von maximal 280°C solite nicht überschritten werden, um eine zu starke Zersetzung von Di-Trimethylolpropan zu vermeiden.

Das über die Kopffraktion abgeführte Di-Trimethylolpropan fällt in einer für technische Anwendungen ausreichenden Qualität an und es können gaschromatographisch ermittelte Wertproduktgehalte von höher als 98 Gew.-% erhalten werden. Auch können Sulfataschegehalte, bestimmt nach DIN 51575, modifiziert unter Zugabe von Schwefelsäure nach dem Abbrennen und vor dem Glühen der Probe, in dem destillativ gereinigten Di-Trimethylolpropan von unter 100 ppm und im Allgemeinen zwischen 15 und 80 ppm erreicht werden.

In einer weiteren Ausgestaltung des erfindungsgemäßen Verfahrens kann vor der destillativen Aufarbeitung des von Feststoffen befreiten Hydrierguts gegebenenfalls eine Behandlung mit einem Ionenaustauscher erfolgen, beispielsweise entweder nur mit einem basischen oder sauren Ionenaustauscher oder kombiniert in beliebiger Reihenfolge. Man arbeitet bei üblichen Temperaturen im Bereich von 1 bis 100°C, vorzugsweise im Bereich von 20 bis 70°C, und bei Normaldruck.

Falls die Hydrierung in Gegenwart gelöster anorganischer Säuren oder niederer organischer Carbonsäuren erfolgte, wird die Lösung nach Abtrennung des Hydrierkatalyators durch Basenzusatz neutral gestellt. Auch in diesem Falle kann sich die Behandlung mit einem Ionenaustauscher anschließen, und zwar bei üblichen Temperaturen im Bereich von 1 bis 100°C, vorzugsweise im Bereich von 20 bis 70°C, und bei Normaldruck. Durch die lonenaustauscherbehandlung werden nicht nur die nach Basenzusatz gebildeten Salze abgetrennt sondern darüber hinaus noch weitere Verunreinigungen.

Zu den basischen Ionenaustauschern zählen solche, die primäre, sekundäre, tertiäre oder quartäre Aminogruppen enthalten. Besondere Bedeutung haben Ionenaustauscher auf Polystyrolbasis, die tertiäre Aminogruppen oder quartäre Aminogruppen in der Basenform enthalten, erlangt. Beispiele für schwach- bis starkbasische Ionenaustauscher sind Amberlit IR 45, Dowex 4 oder Dowex Marathon-A. Besondere technische Bedeutung haben makroretikulare Typen, wie Amberlyst A21, Lewatit MP62, Lewatit MP64, Imac A20, Zerolit G, Amberlit IRA93 oder Amberlyst A26 erlangt.

Schwach oder stark saure Ionenaustauscher enthalten beispielsweise die Carboxylat- oder die Sulfonsäuregruppe, die an eine Polymermatrix auf Basis von Styrol-Divinylbenzol-Copolymeren gebunden sind. Die Carboxylatgruppe kann beispielsweise von aromatischen Carbonsäuren oder aliphatischen Carbonsäuren abgeleitet werden und die Sulfonsäuregruppe von aromatischen oder aliphatischen Sulfonsäuren. Ein stark saurer Ionenaustauscher ist beispielsweise Amberlyst 15, Amberlyst DPT-1 oder Dowex Marathon-C.

Man bringt die Lösung in einem geeigneten Reaktor mit dem Ionenaustauscher in Kontakt. Der Ionenaustauscher kann zum Beispiel in einem Rohrreaktor als Festbett angeordnet werden, durch das die Lösung strömt. Das Festbettvolumen und die Größe der Ionenaustauscherpartikel können in weiten Bereichen variiert und so den gewählten Reaktionsbedingungen und den Verfahrensgegebenheiten, wie der gewünschten Strömungsgeschwindigkeit, angepasst werden. Es hat sich bewährt, Raumgeschwindigkeiten im Bereich von 1 bis 10, insbesondere von 5 bis 8 (V _{Lösung} /[V_{Ionenaustauscher} h]) einzuhalten. Es handelt sich hierbei um Richtgrößen, die zweckmäßig zu wählen sind.

Nach einer anderen Ausführungsform der erfindungsgemäßen Arbeitsweise suspendiert man den Ionenaustauscher, der in diesem Fall sehr feinteilig sein kann, in der Lösung. Es ist zweckmäßig, die Suspension ständig zu bewegen, zum Beispiel durch Rühren oder Einleiten eines Gases, beispielsweise Luft oder Stickstoff, um den innigen Kontakt zwischen der flüssigen Phase und dem Ionenaustauscher zu erzielen. Das Massenverhältnis von flüssiger Phase zu Ionenaustauscher kann weitgehend frei und somit den individuellen Erfordernissen entsprechend eingestellt werden. Es hat sich bewährt, je 100 Gew.-Teile Lösung 1 bis 10, vorzugsweise 3 bis 8 Gew.-Teile Ionenaustauscher einzusetzen. Für die Durchführung dieser Verfahrensvariante eignen sich zum Beispiel Rührkessel oder Autoklaven.

Bei dieser Arbeitsweise unterliegt der Ionenaustauscher jedoch einer mechanischen Belastung und für die Vermischung von flüssiger Phase mit dem Ionenaustauscher sind die Bedingungen so einzustellen, dass ein Abrieb an der Oberfläche der Teilchen oder gar eine mechanische Schädigung der Teilchen vermieden wird.

Die Lösung kann rezirkuliert werden, um durch mehrfache Behandlung der flüssigen Phase die Abtrennung von Verunreinigungen zu vervollständigen. Ebenso ist es möglich, die Adsorption in mehreren Stufen durchzuführen, sowohl absatzweise als auch kontinuierliche Reaktionsführung ist möglich. Die optionale Ionenaustauscherbehandlung eignet sich besonders bei der Aufarbeitung eines wässrigen Hydrierguts.

Nach der Ionenaustauscherbehandlung des flüssigen Hydrierguts wird das erhaltene Eluat destillativ wie zuvor beschrieben in der dreistufigen Schaltung von Destillationseinrichtungen aufgearbeitet. Für die Gewinnung von Di-Trimethylolpropan in ausreichender Qualität ist die optionale Behandlung des flüssigen Hydrierguts mit dem Ionenaustauscher nicht zwingend erforderlich. Dieser zusätzliche Reinigungsschritt kann sich jedoch als zweckmäßig erweisen, wenn die aufzuarbeitenden Nebenströme der Trimethylolpropanherstellung mit einer hohen Salzfracht beladen sind. Salzartige Verunreinigungen lassen sich durch die Behandlung mit dem Ionenaustauscher abtrennen. Sie erweisen sich als störend, da sie bei der nachfolgenden destillativen Aufarbeitung aufgrund der vergleichsweise hohen Sumpftemperaturen die Zersetzung von Di-Trimethylolpropan fördern können. Dabei freigesetzte leichtflüchtige Spaltprodukte wirken sich nachteilig auf die einzustellenden Druckverhältnisse in der Destillation aus, so dass neben der Ausbeute an Di-Trimethylolpropan auch die Qualität leiden kann.

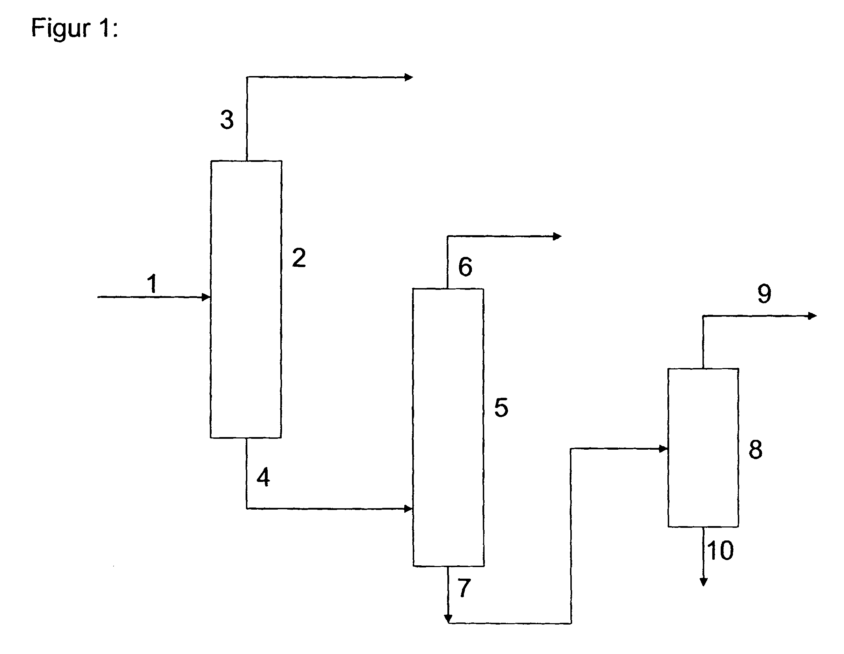

Figur 1 zeigt ein Prinzipschema für die destillative Behandlung des von Feststoffen befreiten flüssigen Hydrierguts, gegebenenfalls nach der Behandlung mit einem Ionenaustauscher. Die über Leitung (1) herangeführte vorzugsweise erhitzte Lösung enthaltend Trimethylolpropan und Di-Trimethylolpropan wird auf eine erste Destillationseinrichtung (2) gegeben, bei der am Kopf über Leitung (3) polares Lösungsmittel und Leichtsieder, beispielsweise Wasser und Methanol, entfernt werden. Über den Sumpf der ersten Destillationseinrichtung (2) wird mit der Leitung (4) die Sumpffraktion abgeführt, in der der Gehalt an dem polaren Lösungsmittel maximal bis 5000 Gew.-ppm, bezogen auf die Masse der Sumpffraktion, beträgt. Bei der ersten Destillationseinrichtung handelt es sich um eine übliche Kolonne mit 2 bis 40 theoretischen Böden. Die Sumpffraktion aus der ersten Destillationseinrichtung (2) wird auf eine zweite Destillationseinrichtung (5) gegeben, die mindestens 5 theoretische Böden aufweist und die als Dünnschichtverdampfer mit Kolonnenaufsatz ausgestaltet ist. Über Leitung (6) wird die Kopffraktion enthaltend überwiegend Trimethylolpropan, Zwischenläufe und Reste von dem polaren Lösungsmittel und Leichtsiedern entnommen und in den Prozess zur Herstellung von Trimethylolpropan zurückgeführt. Die Sumpffraktion aus der zweiten Destillationseinrichtung (5) wird über die Leitung (7) abgeführt und auf die dritte Destillationseinrichtung (8) gegeben. Diese dritte Destillationseinrichtung oder auch Nachlaufabtrenneinrichtung hat mindestens 4, beispielsweise 5 theoretische Böden und ist als Dünnschichtverdampfer mit Kolonnenaufsatz ausgestaltet. Über Leitung (9) wird Di-Trimethylolpropan als Kopffraktion in ausreichender Qualität abgeführt während über Leitung (10) Hochsieder aus dem Verfahren ausgeschleust werden.

Das erfindungsgemäße Verfahren gestattet die wirtschaftliche Verwertung von schwersiedenden Fraktionen und Rückständen, die bei der destillativen Reinigung von Trimethylolpropan anfallen. Durch die Rückführung der daraus gewonnenen trimethylolpropanreichen Produktströme in den gesamten Herstellprozess kann die Anlageneffizienz und die Ausbeute an Trimethylolpropan verbessert werden gegenüber einer Verfahrensführung, bei der die schwersiedenden Fraktionen und Rückstände aus der Trimethylolpropandestillation nicht aufgearbeitet und nicht zurückgeführt werden. Gleichzeitig fällt bei der erfindungsgemäßen Arbeitsweise Di-Trimethylolpropan in einer für technische Anwendungen ausreichenden Qualität an.

In den nachfolgenden Beispielen wird das erfindungsgemäße Verfahren näher beschrieben. Es ist selbstverständlich nicht auf die wiedergegebene Ausführungsform beschränkt.

### Beispiele

### Beispiel 1

Für die erfindungsgemäße Aufarbeitung der schwersiedenden Fraktionen und Rückstände aus der destillativen Reinigung von Trimethylolpropan kam ein Gemisch zum Einsatz, das folgende gaschromatographisch ermittelte Zusammensetzung (%) aufwies:

| | |
|---|---|
| Vorlauf | 0,9 |
| Monocyclisches Formal (I) | 0,1 |
| Trimethylolpropan | 20,8 |
| Zwischenlauf I | 3,3 |
| Di-Trimethylolpropan | 20,2 |
| Zwischenlauf II | 17,8 |
| Lineares bis-Trimethylolpropan-Formal (II) | 30,9 |
| Hochsieder | 6,0 |

Aus dem organischen Rückstand wurde unter Zugabe von Wasser bei 60°C eine homogene wässrige Lösung mit einem Gehalt von 60 Gew.-% aufgelöstem organischen Rückstand hergestellt. Zu 100 Gew.-Teilen wässrige Lösung gab man 5 Gew.-Teile eines handelsüblichen Ruthenium-auf-Aktivkohle-Katalysators in Pulverform und mit einer Metallbeladung von 5 Gew.-% sowie 1,8 Gew.-Teile konzentrierter Schwefelsäure (98%ig). Die erhaltene Suspension wurde anschließend unter folgenden Bedingungen mit Wasserstoff in einem 1 Liter Autoklaven behandelt.

**Tabelle 1: Hydrierung einer wässrigen Lösung von Rückständen aus der Trimethylolpropandestillation an Ruthenium-auf-Aktivkohle in Gegenwart von Schwefelsäure**

| Reaktionsbedingungen | Versuch 1 | Versuch 2 | Versuch 3 | Versuch 4 (Vergleich) |
|---|---|---|---|---|
| Temperatur (°C) | 200 | 200 | 200 | 130 |
| Druck (MPa) | 4 | 8 | 20 | 20 |
| Reaktionsdauer (h) | 5 | 5 | 5 | 5 |
| Gaschromatographisch ermittelte Zusammensetzung (%; organischer Anteil, wasserfrei): | | | | |
| Vorlauf | 5,2 | 4,7 | 7,2 | 1,1 |
| Monocycl. Formal (I) | 0,2 | 0,1 | 0,1 | 20,2 |
| Trimethylolpropan | 74,4 | 72,7 | 75,6 | 56,6 |
| Zwischenlauf I | 4,2 | 1,4 | 1,0 | 5,3 |
| Di-Trimethylolpropan | 15,5 | 20,8 | 15,8 | 16,3 |
| Zwischenlauf II | 0,1 | 0,1 | 0,1 | 0,2 |
| Lineares bis- Trimethylolpropan-Formal (II) | 0,1 | 0,1 | 0,1 | 0,1 |
| Hochsieder | 0,3 | 0,1 | 0,1 | 0,2 |

### Beispiel 2

Für die erfindungsgemäße Aufarbeitung der schwersiedenden Fraktionen und Rückstände aus der destillativen Reinigung von Trimethylolpropan kam ein Gemisch zum Einsatz, das folgende gaschromatographisch ermittelte Zusammensetzung (%) aufwies:

| | |
|---|---|
| Vorlauf | 0 |
| Monocyclisches Formal (I) | 0 |
| Trimethylolpropan | 7,0 |
| Zwischenlauf I | 19,8 |
| Di-Trimethylolpropan | 20,3 |
| Zwischenlauf II | 0,1 |
| Lineares bis-Trimethylolpropan-Formal (II) | 47,0 |
| Hochsieder | 5,8 |

Aus dem organischen Rückstand wurde unter Zugabe von Wasser bei 60°C eine homogene wässrige Lösung mit einem Gehalt von 60 Gew.-% aufgelöstem organischen Rückstand hergestellt. Zu 100 Gew.-Teilen wässrige Lösung gab man 0,5 Gew.-Teile eines handelsüblichen Ruthenium-auf-Aktivkohle-Katalysators in Pulverform und mit einer Metallbeladung von 5 Gew.-%, sowie 0,5 Gew.-Teile eines sauren, handelsüblichen Zeoliths vom Y-Typ. Die erhaltene Suspension wurde anschließend unter folgenden Bedingungen mit Wasserstoff in einem 1 Liter Autoklaven behandelt.

**Tabelle 2: Hydrierung einer wässrigen Lösung von Rückständen aus der Trimethylolpropandestillation an Ruthenium-auf-Aktivkohle in Gegenwart von Zeolith vom Y-Typ**

| Reaktionsbedingun gen | Versuch 5 | Versuch 6 | Versuch 7 | Versuch 8 | Versuch 9 |
|---|---|---|---|---|---|
| Temperatur (°C) | 190 | 200 | 210 | 220 | 230 |
| Druck (MPa) | 13 | 13 | 13 | 13 | 13 |
| Reaktionsdauer (h) | 3 | 3 | 3 | 3 | 3 |
| Gaschromatographisch ermittelte Zusammensetzung (%; organischer Anteil, wasserfrei): | | | | | |
| Vorlauf | 3,6 | 6,0 | 6,6 | 5,8 | 9,4 |
| Monocycl. Formal (I) | 10,3 | 5,4 | 1,7 | 0,4 | 0,1 |
| Trimethylolpropan | 60,2 | 63,1 | 65,9 | 68,4 | 64,4 |
| Zwischenlauf I | 3,7 | 2,4 | 1,9 | 1,4 | 1,6 |
| Di-Trimethylolpropan | 21,7 | 22,7 | 23,5 | 23,6 | 24,2 |
| Zwischenlauf II | 0,1 | 0,1 | 0,1 | 0,1 | 0,1 |
| Lineares bis- Trimethylolpropan-Formal (II) | 0,1 | 0,1 | 0,1 | 0,1 | 0,1 |
| Hochsieder | 0,3 | 0,2 | 0,2 | 0,2 | 0,1 |

### Beispiel 3

Für die destillative Aufarbeitung des nach Filtration des Katalysators und des sauren Zeoliths gemäß Beispiel 2 erhaltenen Hydrierguts kam eine wässrige Lösung zum Einsatz, die 40 Gew.-% Wasser und 60 Gew.-% organische Anteile enthielt, wobei der organische Anteil folgende gaschromatographisch ermittelte Zusammensetzung (%) aufwies:

| | |
|---|---|
| Vorlauf | 6,3 |
| Monocyclisches Formal (I) | 0,3 |
| Trimethylolpropan | 64,4 |
| Zwischenlauf I | 0,6 |
| Di-Trimethylolpropan | 28,0 |
| Zwischenlauf II | 0,1 |
| Lineares bis-Trimethylolpropan-Formal (II) | 0,1 |
| Hochsieder | 0,2 |

### Beispiel 3a (1): Wasser-, Vorlaufentfernung

In einer ersten Destillation wurden an einer 20-Böden-Kolonne mit Sumpfblase bei einer Sumpftemperatur von 96°C und bei einem Druck von 73 mbar hPa Wasser und Leichtsieder als Destillat entfernt. Der erhaltene Destillationssumpf enthielt etwa 800 Gew.-ppm Wasser und wies folgende gaschromatographisch ermittelte Zusammensetzung (%) auf:

| | |
|---|---|
| Vorlauf | 0,6 |
| Monocyclisches Formal (I) | 0,1 |
| Trimethylolpropan | 72,0 |
| Zwischenlauf I | 0,8 |
| Di-Trimethylolpropan | 26,0 |
| Zwischenlauf II | 0,1 |
| Lineares bis-Trimethylolpropan-Formal (II) | 0,1 |
| Hochsieder | 0,3 |

Beispiel 3a (2): Leichtsiederentfernung, Trimethylolpropanabreicherung Das Sumpfprodukt gemäß Beispiel 3a (1) wurde einer erneuten Destillation an einer 20-Böden-Kolonne mit Sumpfblase unterzogen. Der angelegte Druck betrug 3 hPa bei einer Sumpftemperatur von 255°C. Es wurde ein Rücklaufverhältnis von 1:1 eingestellt. Das erhaltene Sumpfprodukt wies folgende gaschromatographisch ermittelte Zusammensetzung (%) auf:

| | |
|---|---|
| Vorlauf | 0,1 |
| Monocyclisches Formal (I) | 0,0 |
| Trimethylolpropan | 2,4 |
| Zwischenlauf I | 1,6 |
| Di-Trimethylolpropan | 93,4 |
| Zwischenlauf II | 0,8 |
| Lineares bis-Trimethylolpropan-Formal (II) | 0,0 |
| Hochsieder | 1,7 |

### Beispiel 3b: Entfernung von Trimethylolpropan angereicherten Produktströmen

Das Sumpfprodukt aus der ersten Destillation gemäß Beispiel 3a (1) (70/30-Mischung) wurde für die zweite Destillation eingesetzt. Die zweite Destillation wurde so ausgestaltet, dass Zwischenlauffraktionen im Destillationssumpf möglichst abgereichert waren. In der Tabelle 3 sind unterschiedliche Ausführungsformen für die zweite Destillation zusammengestellt. Die gaschromatographische Analyse gibt die Zusammensetzung (%) des Einsatzes sowie die Zusammensetzung des Destillationssumpfes wieder.

**Tabelle 3: Entfernung von mit Trimethylolpropan angereicherten ProduktStrömen aus dem Destillationssumpf gemäß Beispiel 3a (1) [Einsatz: 70/30-Mischung]; gaschromatographische Analyse der Sumpfprodukte**

| | | **3b (1)** | **3b (2)** | **3b (3) Vergleich** |
|---|---|---|---|---|
| | Einsatz (70/30-Mischung) | Dünnschichtverdampfer mit Füllkörperkolonne | Dünnschichtverdampfer mit Packungskolonne | Nur Packungskolonne mit Sumpfblase |
| Temperaturen | | | | |
| Kopf | [°C] | 160 | 163 | 165 |
| Seite | [°C] | - | 242 | 190 |
| Mantel / Sumpf | [°C] | 270 | 265 | 269 |
| Kolonnenkopf | [hPa] | 5 | 5 | 4 |
| Differenzdruck | [hPa] | 29 | 11 | 17 |
| Rücklaufverhältnis | | 1/3 | ohne | ohne |
| Kopfabnahme | [%] | 76 | 75,3 | 77 |
| Sumpfabnahme | [%] | 24 | 24,7 | 23 |
| Bodenzahl | | 11 | 15 | 15 |
| Verweilzeit | [s] | 10-30 | 10-30 | 3-5 Stunden |
| | | | | |
| Gaschromatographische Zusammensetzung (%): | | | | |
| Vorlauf | 0,6 | 0,1 | 0,1 | 0,1 |
| Monocyclisches Formal (I) | 0,1 | 0,0 | 0,0 | 0,0 |
| Trimethylolpropan | 72,0 | 0,1 | 0,3 | 0,1 |
| Zwischenlauf I | 0,8 | 1,5 | 0,4 | 0,9 |
| Di-Trimethylolpropan | 26,0 | 97,3 | 98,1 | 97,5 |
| Zwischenlauf II | 0,1 | 0,1 | 0,1 | 0,1 |
| Lineares bis-Trimethylol-propan -Formal (II) | 0,1 | 0,1 | 0,0 | 0,1 |
| Hochsiede.r | 0,3 | 0,8 | 1,0 | 1,2 |

Wie das Beispiel 3b (2) zeigt, kann durch den Einsatz einer Packungskolonne, beispielsweise durch eine Kolonne mit einem Durchmesser von 40 mm und ausgestattet mit einer Montz-Packung der Zwischenlauf I im Sumpf abgereichert werden. Bei Beispiel 3b (3) ist mit vergleichsweise hohen Verweilzeiten zu arbeiten, so dass es bei den hohen Destillationstemperaturen zu Zersetzungen unter Bildung flüchtiger Verbindungen kommt und ein vergleichsweise hoher Differenzdruck gegenüber Beispiel 3b (2), das mit dem gleichen Kolonnentyp arbeitet, während der Destillation beobachtet wird. Dennoch wird auch bei dieser Ausgestaltung der zweiten Destillation ein Sumpfprodukt mit einem Gehalt von Di-Trimethylolpropan von 97,5% erhalten.

Der Einsatz von einer Packungskolonne mit einer höheren Anzahl an Trennstufen ist aber einer Füllkörperkolonne vorzuziehen.

Für die folgenden Destillationsversuche 3b (4) - 3b (6) wurde das Sumpfprodukt aus der Leichtsiederabtrennung und Trimethylolpropanabreicherung gemäß Beispiel 3a (2) (93%ige Ware) verwendet. Die Bedingungen der zweiten Destillation sowie die gaschromatographische Analyse der Sumpfprodukte (%) sind in der folgenden Tabelle 4 zusammengestellt.

**Tabelle 4: Entfernung von mit Trimethylolpropan angereicherten Produktströmen aus dem Sumpfprodukt gemäß Beispiel 3a (2) (93%ige Ware, Einsatz) gaschromatographische Analyse der Sumpfprodukte**

| | | **3b (4)** | **3b (5) Vergleich** | **3b (6) Vergleich** |
|---|---|---|---|---|
| | Einsatz (93%ige Ware) | Dünnschichtverdampfer mit Packungskolonne | Nur Füllkörperkolonne mit Sumpfblase | Nur Kurzwegverdampfer ohne Kolonne |
| Temperaturen | | | | |
| Kopf | [°C] | 145-175 | 144-215 | 185-190 |
| Seite | [°C] | 149-180 | 148-223 | - |
| Mantel/Sumpf | [°C] | 260 | 270 | 200 |
| Kolonnenkopf | [hPa] | 1-2 | 4 | 3 |
| Differenzdruck | [hPa] | 8 | 16 | - |
| Rücklaufverhältnis | | 1/2 | 2/1 | ohne |
| Kopfabnahme | [%] | 24,3 | 13,1 | 50 |
| Sumpfabnahme | [%] | 75,7 | 86,9 | 50 |
| Bodenzahl | | 5 | 15 | 1 |
| Verweilzeit | [s] | 10-30 | 3-5 Stunden | 5-20 |
| | | | | |
| Gaschromatographische Zusammensetzung (%): | | | | |
| Vorlauf | 0,1 | 0,1 | 0,1 | 0,0 |
| Monocyclisches Formal (I) | 0,0 | 0,0 | 0,0 | 0,0 |
| Trimethylolpropan | 2,4 | 0,3 | 0,1 | 2,3 |
| Zwischenlauf I | 1,6 | 0,7 | 0,7 | 2,3 |
| Di-Trimethylolpropan | 93,4 | 97,7 | 96,7 | 94,1 |
| Zwischenlauf II | 0,8 | 0,1 | 0,1 | 0,2 |
| Lineares bis-Trimethylolpropan -Formal (II) | 0,0 | 0,0 | 0,0 | 0,0 |
| Hochsieder | 1,7 | 1,1 | 2,3 | 1,1 |

Wie das Vergleichsbeispiel 3b (6) zeigt, gelingt die Abreicherung des Zwischenlaufs I im Sumpfprodukt der zweiten Destillation nicht, wenn ohne Kolonnenaufsatz und nur mit einem Kurzwegverdampfer gearbeitet wird. Für die Abreicherung des Zwischenlaufs I ist gemäß Beispiel 3b (4) ein Kolonnenaufsatz mit 5 theoretischen Böden notwendig, selbst wenn der Destillationseinsatz bereits einen hohen Gehalt an Di-Trimethylolpropan aufweist.

### Beispiel 3c: Nachlaufabtrennung

Das gemäß Beispiel 3b (3) erhaltene Sumpfprodukt wurde für die dritte Destillation zur Nachlaufabtrennung eingesetzt. Das gewünschte Di-Trimethylolpropan wurde als Kopfprodukt in ausreichender Qualität erhalten. Die Destillationsbedingungen sowie die gaschromatographische Analyse (%) des Destillats sind in der Tabelle 5 angegeben.

**Tabelle 5: Nachlaufabtrennung aus dem Sumpfprodukt gemäß Beispiel 3b (3), gaschromatographische Analyse (%) des Destillats**

| | | **3c (7)** | **3c (8)** | **3c (9)** |
|---|---|---|---|---|
| | | | **Verpleich** | |
| | Einsatz Beispiel 3b (3) | Dünnschichtverdampfer mit Packungskolonne | Nur Füllkörperkolonne mit Sumpfblase | Dünnschichtverdampfer mit Packungskolonne |
| Temperaturen | | | | |
| Kopf | [°C] | 222 | 235 | 135 |
| Seite | [°C] | 230 | 240 | 160 |
| Sumpf | [°C] | 265 | 290-70 | 265 |
| Kolonnenkopf | [hPa] | 3 | 5 | 0,3 |
| Differenzdruck | [hPa] | 10 | 30-35 | - |
| Rücklaufverhältnis | | ohne | ohne | ohne |
| Kopfabnahme | [%] | 90,1 | 76,2 | 95,4 |
| Sumpfabnahme | [%] | 9,9 | 23,8 | 4,6 |
| Bodenzahl | | 15 | 15 | 15 |
| Verweilzeit | [s] | 5-8 | 3-5 Stunden | 5-9 |
| | | | | |
| Gaschromatographische Zusammensetzung (%): | | | | |
| Vorlauf | 0,1 | 0,0 | 11,5 | 0,0 |
| Monocyclisches Formal (I) | 0,0 | 0,0 | 0,0 | 0,0 |
| Trimethylolpropan | 0,1 | 0,6 | 14,8 | 0,5 |
| Zwischenlauf I | 0,9 | 0,1 | 2,2 | 0,1 |
| Di-Trimethylolpropan | 97,5 | 98,6 | 70,7 | 98,5 |
| Zwischenlauf II | 0,1 | 0,5 | 0,3 | 0,7 |
| Lineares bis-Trimethylolpropan -Formal (II) | 0,1 | 0,0 | 0,1 | 0,1 |
| Hochsieder | 1,2 | 0,2 | 0,4 | 0,1 |
| | | | | |
| Aschewert DIN 51575, modifiziert | | <50 ppm | - | <50 ppm |
| Gardner Farbzahl ASTM D 1544 | >6 | 1 | 1 | 1 |

Wie der Vergleich der Beispiele 3c (7) und 3c (9) mit Vergleichsbeispiel 3c (8) zeigt, ist die Verwendung eines Dünnschichtverdampfers mit Kolonnenaufsatz erforderlich um Di-Trimethylolpropan als Kopfprodukt in ausreichender Qualität zu erhalten. Eine Destillationseinrichtung aus Sumpfblase mit Kolonnenaufsatz ist für die Nachlaufabtrennung nicht geeignet, da es bei dieser Anordnung aufgrund der hohen Temperaturen und langen Verweilzeiten zu einer vermehrten Zersetzung, angezeigt durch eine deutliche Vorlaufbildung und Abnahme des Di-Trimethylolpropangehaltes, kommt.

## Patentansprüche

1. Verfahren zur Gewinnung von Di-Trimethylolpropan und mit Trimethylolpropan angereicherten Produktströmen aus den schwersiedenden Fraktionen und Rückständen, die bei der destillativen Reinigung von Trimethylolpropan anfallen, **dadurch gekennzeichnet, dass** man:
(a) diese schwersiedenden Fraktionen und Rückstände vereinigt und unter Zusatz eines polaren Lösungsmittels eine Lösung herstellt;
(b) die nach Schritt a) hergestellte Lösung bei einer Temperatur von 160 bis 280°C und bei einem Druck von 1 bis 30 MPa mit Wasserstoff in Gegenwart eines Katalysators und einer aciden Verbindung behandelt;
(c) die nach Schritt b) erhaltene Lösung vom Katalysator und weiteren Feststoffen, falls vorhanden, abtrennt;
(d) die nach Schritt c) erhaltene Lösung in einer ersten Destillationseinrichtung in eine Kopffraktion enthaltend das polare Lösungsmittel und Leichtsieder und in eine Sumpffraktion mit einem Gehalt an dem polaren Lösungsmittel bis 5000 Gew.-ppm, bezogen auf die Sumpffraktion, auftrennt;
(e) die nach Schritt d) erhaltene Sumpffraktion auf eine zweite Destillationseinrichtung mit mindestens 5 theoretischen Böden, die als Dünnschichtverdampfer mit Kolonnenaufsatz ausgestaltet ist, gibt und eine mit Trimethylolpropan angereicherte Kopffraktion abzieht und eine Sumpffraktion entnimmt; und
(f) die nach Schritt e) erhaltene Sumpffraktion auf eine dritte Destillationseinrichtung mit mindestens 4 theoretischen Böden, die als Dünnschichtverdampfer mit Kolonhenaufsatz ausgestaltet ist, gibt, in der Di-Trimethylolpropan als Kopffraktion gewonnen wird und Hochsieder als Sumpffraktion entfernt werden.

2. Verfahren gemäß Anspruch 1, **dadurch gekennzeichnet, dass** die gemäß Schritt d) erhaltene Sumpffraktion einen Gehalt an dem polaren Lösungsmittel bis 1000 Gew.-ppm und insbesondere bis 500 Gew.-ppm, bezogen auf die Sumpffraktion, aufweist.

3. Verfahren gemäß Anspruch 1 oder 2, **dadurch gekennzeichnet, dass** die zweite Destillationseinrichtung mindestens 8 theoretische Böden aufweist.

4. Verfahren gemäß einem oder mehreren der Ansprüche 1 bis 3,
**dadurch gekennzeichnet, dass** in Schritt e) der Kolonnenaufsatz als Füllkörperkolonne oder Packungskolonne ausgestaltet ist.

5. Verfahren gemäß einem oder mehreren der Ansprüche 1 bis 4,
**dadurch gekennzeichnet, dass** die zweite Destillationseinrichtung bei einer Temperatur von 210 bis 280°C und bei einem Druck von 2 bis 10 hPa betrieben wird.

6. Verfahren gemäß einem oder mehreren der Ansprüche 1 bis 5,
**dadurch gekennzeichnet, dass** die zweite Destillationseinrichtung bei einer Verweilzeit von 2 bis 60 Sekunden betrieben wird.

7. Verfahren gemäß einem oder mehreren der Ansprüche 1 bis 6,
**dadurch gekennzeichnet, dass** die dritte Destillationseinrichtung 4 bis 20 theoretische Böden aufweist.

8. Verfahren gemäß einem oder mehreren der Ansprüche 1 bis 7,
**dadurch gekennzeichnet, dass** in Schritt f) der Kolonnenaufsatz als Füllkörperkolonne oder Packungskolonne ausgestaltet ist.

9. Verfahren gemäß einem oder mehreren der Ansprüche 1 bis 8,
**dadurch gekennzeichnet, dass** die dritte Destillationseinrichtung bei einer Temperatur von 240 bis 280°C und bei einem Druck von 0,2 bis 5 hPa betrieben wird.

10. Verfahren gemäß einem oder mehreren der Ansprüche 1 bis 9,
**dadurch gekennzeichnet, dass** in der dritten Destillationseinrichtung die Verweilzeit der Kopffraktion 1 bis 30 Sekunden beträgt.

11. Verfahren gemäß einem oder mehreren der Ansprüche 1 bis 10,
**dadurch gekennzeichnet, dass** nach dem Schritt c) und vor dem Schritt d) die nach Schritt c) erhaltene Lösung mit einem Ionenaustauscher behandelt wird.

12. Verfahren gemäß Anspruch 11, **dadurch gekennzeichnet, dass** die nach Schritt c) erhaltene Lösung sowohl mit einem basischen als auch mit einem sauren Ionenaustauscher in beliebiger Reihenfolge behandelt wird.

13. Verfahren gemäß einem oder mehreren der Ansprüche 1 bis 12,
**dadurch gekennzeichnet, dass** man als polares Lösungsmittel einen C₁-C₅-aliphatischen Alkohol, einen C₂-C₁₀-Dialkylether oder Wasser verwendet.

## Claims

1. Process for obtaining ditrimethylolpropane and trimethylolpropane-enriched product streams from the high-boiling fractions and residues which are obtained in the distillative purification of trimethylolpropane, **characterized in that**:
(a) these high-boiling fractions and residues are combined and a polar solvent is added to produce a solution;
(b) the solution produced according to step a) is treated at a temperature of 160 to 280°C and at a pressure of 1 to 30 MPa with hydrogen in the presence of a catalyst and of an acidic compound;
(c) the solution obtained according to step b) is removed from the catalyst and further solids, if present;
(d) the solution obtained according to step c) is separated in a first distillation unit into a tops fraction comprising the polar solvent and low boilers and into a bottoms fraction with a content of the polar solvent up to 5000 ppm by weight, based on the bottoms fraction;
(e) the bottoms fraction obtained according to step d) is supplied to a second distillation unit with at least 5 theoretical plates, said unit being configured as a thin-film evaporator with a column attachment, and a trimethylolpropane-enriched tops fraction is drawn off and a bottoms fraction is withdrawn; and
(f) the bottoms fraction obtained according to step e) is supplied to a third distillation unit with at least 4 theoretical plates, said unit being configured as a thin-film evaporator with a column attachment, in which ditrimethylolpropane is obtained as the tops fraction and high boilers are removed as the bottoms fraction.

2. Process according to Claim 1, **characterized in that** the bottoms fraction obtained according to step d) has a content of the polar solvent up to 1000 ppm by weight and especially up to 500 ppm by weight, based on the bottoms fraction.

3. Process according to Claim 1 or 2, **characterized in that** the second distillation unit has at least 8 theoretical plates.

4. Process according to one or more of Claims 1 to 3, **characterized in that**, in step e), the column attachment is configured as a column with random packing or column with structured packing.

5. Process according to one or more of Claims 1 to 4, **characterized in that** the second distillation unit is operated at a temperature of 210 to 280°C and at a pressure of 2 to 10 hPa.

6. Process according to one or more of Claims 1 to 5, **characterized in that** the second distillation unit is operated with a residence time of 2 to 60 seconds.

7. Process according to one or more of Claims 1 to 6, **characterized in that** the third distillation unit has 4 to 20 theoretical plates.

8. Process according to one or more of Claims 1 to 7, **characterized in that**, in step f), the column attachment is configured as a column with random packing or column with structured packing.

9. Process according to one or more of Claims 1 to 8, **characterized in that** the third distillation unit is operated at a temperature of 240 to 280°C and at a pressure of 0.2 to 5 hPa.

10. Process according to one or more of Claims 1 to 9, **characterized in that** the residence time of the tops fraction in the third distillation unit is 1 to 30 seconds.

11. Process according to one or more of Claims 1 to 10, **characterized in that** the solution obtained according to step c), after step c) and before step d), is treated with an ion exchanger.

12. Process according to Claim 11, **characterized in that** the solution obtained according to step c) is treated both with a basic and with an acidic ion exchanger in any sequence.

13. Process according to one or more of Claims 1 to 12, **characterized in that** the polar solvent used is a C₁-C₅ aliphatic alcohol, a C₂-C₁₀ dialkylether or water.

## Revendications

1. Procédé pour la production de ditriméthylolpropane et de flux de produits enrichis en triméthylolpropane à partir des fractions lourdes et de résidus produits lors de la purification par distillation de triméthylolpropane, **caractérisé en ce qu'**on
(a) rassemble ces fractions lourdes et ces résidus et prépare une solution avec addition d'un solvant polaire ;
(b) traite la solution préparée selon l'étape a) à une température de 160 à 280°C et à une pression de 1 à 30 MPa avec de l'hydrogène en présence d'un catalyseur et d'un composé acide ;
(c) sépare la solution obtenue selon l'étape b) du catalyseur et d'autres solides, s'ils sont présents ;
(d) sépare la solution obtenue selon l'étape c) dans un premier dispositif de distillation en une fraction de tête contenant le solvant polaire et les substances à bas point d'ébullition et en une fraction de fond présentant une teneur en solvant polaire jusqu'à 5000 ppm en poids par rapport à la fraction de fond ;
(e) introduit la fraction de fond obtenue selon l'étape d) sur un deuxième dispositif de distillation présentant au moins 5 plateaux théoriques, qui est conçu comme évaporateur à couches minces présentant un adaptateur de colonne, et on soutire une fraction de tête enrichie en triméthylolpropane et on prélève une fraction de fond ; et
(f) introduit la fraction de fond obtenue selon l'étape e) sur un troisième dispositif de distillation présentant au moins 4 plateaux théoriques, qui est conçu comme évaporateur à couches minces présentant un adaptateur de colonne, dans lequel on obtient le ditriméthylolpropane comme fraction de tête et des substances à point d'ébullition élevé sont éliminées en tant que fraction de fond.

2. Procédé selon la revendication 1, **caractérisé en ce que** la fraction de fond obtenue selon l'étape d) présente une teneur en solvant polaire de jusqu'à 1000 ppm en poids et en particulier de jusqu'à 500 ppm en poids, par rapport à la fraction de fond.

3. Procédé selon la revendication 1 ou 2, **caractérisé en ce que** le deuxième dispositif de distillation présente au moins 8 plateaux théoriques.

4. Procédé selon l'une ou plusieurs des revendications 1 à 3, **caractérisé en ce que**, dans l'étape e), l'adaptateur de colonne est conçu comme une colonne à corps de remplissage ou comme une colonne garnie.

5. Procédé selon l'une ou plusieurs des revendications 1 à 4, **caractérisé en ce que** le deuxième dispositif de distillation est exploité à une température de 210 à 280°C et à une pression de 2 à 10 hPa.

6. Procédé selon l'une ou plusieurs des revendications 1 à 5, **caractérisé en ce que** le deuxième dispositif de distillation est exploité à un temps de séjour de 2 à 60 secondes.

7. Procédé selon l'une ou plusieurs des revendications 1 à 6, **caractérisé en ce que** le troisième dispositif de distillation présente 4 à 20 plateaux théoriques.

8. Procédé selon l'une ou plusieurs des revendications 1 à 7, **caractérisé en ce que**, dans l'étape f), l'adaptateur de colonne est conçu comme une colonne à corps de remplissage ou comme une colonne garnie.

9. Procédé selon l'une ou plusieurs des revendications 1 à 8, **caractérisé en ce que** le troisième dispositif de distillation est exploité à une température de 240 à 280°C et à une pression de 0,2 à 5 hPa.

10. Procédé selon l'une ou plusieurs des revendications 1 à 9, **caractérisé en ce que**, dans le troisième dispositif de distillation, le temps de séjour de la fraction de tête est de 1 à 30 secondes.

11. Procédé selon l'une ou plusieurs des revendications 1 à 10, **caractérisé en ce que** la solution obtenue selon l'étape c) est traitée, après l'étape c) et avant l'étape d), par un échangeur d'ions.

12. Procédé selon la revendication 11, **caractérisé en ce que** la solution obtenue selon l'étape c) est traitée, tant avec un échangeur d'ions basique qu'avec un échangeur d'ions acide, dans un ordre quelconque.

13. Procédé selon l'une ou plusieurs des revendications 1 à 12, **caractérisé en ce qu'**on utilise, comme solvant polaire, un alcool aliphatique en C₁-C₅, un C₂-C₁₀-dialkyléther ou de l'eau.
